# EUROPEAN PATENT SPECIFICATION

(11) **EP 1 528 108 B1**
(45) Date of publication and mention of the grant of the patent: **15.08.2007**
(21) Application number: 04025952.5
(22) Date of filing: 02.11.2004
(51) Int. Cl.: C12N 1/21, C12P 13/12

(54) **Method for producing l-cysteine using bacteria belonging to the genus Escherichia**
Verfahren zur Herstellung von L-Cystein unter Verwendung eines zur Gattung Escherichia gehörenden Bakteriums
Procédé de production de L-Cystéine à l'aide d'une bactérie appartenante au genre Escherichia

(30) Priority: 03.11.2003 RU 2003131993
(43) Date of publication of application: 04.05.2005
(73) Proprietor: Ajinomoto Co., Inc., Tokyo 104-8315 (JP)
(72) Inventor: Ziyatdinov, Mikhail Kharisovich, 115561 Moscow (RU); Redkina, Ekaterina Igorevna, 113208 Moscow (RU); Gusyatiner, Mikhail Markovich, 113646 Moscow (RU)
(74) Representative: Strehl Schübel-Hopf & Partner

(56) References cited:
- EP-A- 1 298 200
- WO-A-03/006666
- DE-A1- 10 136 986
- DE-C1- 19 949 579
- NEIDHARDT F.C.: "Escherichia coli and Salmonella, 2nd edition" 1996, ASM PRESS , WASHINGTON DC , XP002317084 especially figure 1B and table 3 * page 514 - page 527 *
- SIRKO A ET AL: "SULFATE AND THIOSULFATE TRANSPORT IN ESCHERICHIA-COLI K-12 NUCLEOTIDE SEQUENCE AND EXPRESSION OF THE CYS-TWAM GENE CLUSTER" JOURNAL OF BACTERIOLOGY, vol. 172, no. 6, 1990, pages 3351-3357, XP008042789 ISSN: 0021-9193
- KREDICH N M: "THE MOLECULAR BASIS FOR POSITIVE REGULATION OF CYS PROMOTERS IN SALMONELLA TYPHIMURIUM AND ESCHERICHIA COLI" DIAGNOSTIC MOLECULAR MICROBIOLOGY : PRINCIPLES AND APPLICATIONS, XX, XX, vol. 6, no. 19, October 1992 (1992-10), pages 2747-2759, XP001013130

## Description

### BACKGROUND OF THE INVENTION

### Field of the invention

The present invention relates to biotechnology, and specifically to a method for producing L-cysteine by fermentation. The present invention specifically relates to genes derived from *Escherichia coli.* These genes have been found to be useful for improving L-cysteine production of the *E. coli.*

### Description of the Related Art

Conventionally, L-amino acids have been industrially produced by utilizing strains of microorganisms obtained from natural sources or mutants thereof which have been specifically modified to enhance L-amino acid production.

Many techniques have been reported regarding enhancement of L-amino acid production, for example, by transformation of a microorganism by recombinant DNA (see, for example, US patent No. 4,278,765). These techniques are based on increasing the activities of the enzymes involved in amino acid biosynthesis and/or desensitizing the target enzymes from the feedback inhibition by the produced L-amino acid (see, for example, US patent Nos. 5,661,012 and 6,040,160).

The synthesis of L-cysteine from inorganic sulfur is the predominant mechanism by which reduced sulfur is incorporated into organic compounds in microorganisms, including *Salmonella* and *Escherichia coli.* In this process, inorganic sulfate, the most abundant source of utilizable sulfur in the aerobic biosphere, is taken up and reduced to sulfide, which is then incorporated into L-cysteine in a step that is equivalent to the fixation of ammonia into glutamine or glutamate. Sulfate uptake is performed by sulphate permease, which is encoded by the *cysTWA* and *sbp* (sulphate binding protein) genes. Two additional mechanisms for sulfur fixation have been described for *S. typhimurium* and *E. coli.* The first mechanism occurs through the reaction of thiosulfate with O-acetyl-L-serine catalyzed by O-acetylserine(thiol)-lyase-B encoded by the *cysM* gene to form the thiosulfonate S-sulfocysteine, which is then reduced to L-cysteine. In this mechanism thiosulphate is transported into the cell by thiosulphate permease, which is encoded by the *cysPTWA* genes. As alternative, sulfide could be incorporated into O-acetyl-L-serine through the reaction catalyzed by O-acetylserine(thiol)-lyase-A or B, which are encoded by *cysK* and *cysM* genes, respectively, yielding L-cysteine. The second mechanism involves the reaction of O-succinyl-L-homoserine with sulfide to form homocysteine in a reaction catalyzed by cystathionine γ-synthase. (Escherichia coli and Salmonella, Second Edition, Editor in Chief: F.C.Neidhardt, ASM Press, Washington D.C., 1996).

A process for the preparation of L-threonine by fermentation of microorganisms of the *Enterobacteriaceae* family in which at least one or more of the genes of cysteine biosynthesis chosen from *cysG, cysB, cysZ, cysK, cysM, cysA, cysW, cysU, cysP, cysD, cysN, cysC, cysJ, cysI, cysH, cysE* and *sbp* is (are) enhanced, in particular over-expressed, has been disclosed (WO03006666A2).

There have been no reports to date, however, describing an improvement in L-cysteine productivity by a bacterium grown in a thiosulphate-containing medium, and wherein the bacterium has been modified to have enhanced expression of the *cysPTWAM* cluster.

### SUMMARY OF THE INVENTION

An object of the present invention is to enhance the productivity of L-cysteine-producing bacterial strains. It is a further object of the present invention to provide a method for producing the L-cysteine using such strains.

It is a further object of the present invention to provide an L-cysteine producing bacterium belonging to the genus *Escherichia,* wherein the bacterium has been modified so that the expression amount of the *cysPTWAM* cluster genes is greater than that of a strain before modification by increasing the copy number of the *cysPTWAM* cluster genes or modifying an expression control sequence of said genes.

It is a further object of the present invention to provide the bacterium as described above, wherein the copy number is increased by transforming the bacterium with a multi-copy vector harboring the *cysPTWAM* cluster genes.

It is a further object of the present invention to provide the bacterium as described above, wherein the native promoter of said cluster is replaced with a promoter which is more patent than the native promoter.

It is a further object of the present invention to provide the bacterium as described above, wherein the copy number is increased by integrating additional copies of the *cysPTWAM* cluster genes into chromosome of the bacterium.

It is a further object of the present invention to provide the bacterium as described above, wherein the *cysPTWAM* cluster genes are derived from a bacterium belonging to the genus *Escherichia.*

It is a further object of the present invention to provide the bacterium as described above, wherein the bacterium is further modified so that the expression amount of the *ydeD* open reading frame is greater than that of a strain before modification.

It is a still further object of the present invention to provide a method for producing L-cysteine, which comprises cultivating the bacterium as described above in a culture medium containing thiosulphate and collecting L-cysteine from the culture medium.

It is a further object of the present invention to provide the method as described above, wherein the expression amount of L-cysteine biosynthesis genes in the bacterium is greater than that of a strain before modification.

### BRIEF EXPLANATION OF THE DRAWINGS

Figure 1 shows the relative position of primers cysEF, cysEX-1, cysEX-2 and cysER.
Figure 2 shows the structure of plasmid pACYC-DES.
Figure 3 shows the structure of plasmid pMW119int.

### DETAILED DESCRIPTION OF THE INVENTION

The aforementioned objects were achieved by the discovery that enhancing the expression of the *cysPTWA* cluster genes, as well as the *cysM* gene, which encode a system for sulphate/thiosulphate transport and O-acetylserine(thiol)-lyase-B, respectively, can enhance L-cysteine production when the novel modified strain described herein is cultivated in a medium containing thiosulphate.

The bacterium of the present invention is an L-cysteine-producing bacterium belonging to the genus *Escherichia,* which has enhanced expression of the genes of *cysPTWAM* cluster, which enhances or increases the production yield of L-cysteine. More specifically, the bacterium of the present invention is an L-cysteine-producing bacterium belonging to the genus *Escherichia,* wherein the bacterium has been modified to enhance expression of the *cysPTWAM* cluster genes.

"L-cysteine-producing bacterium" means a bacterium, which has an ability to produce and secrete L-cysteine into a medium, when the bacterium is cultured in the medium The term "L-cysteine-producing bacterium" as used herein may also mean a bacterium, which is able to produce and secrete L-cysteine into a culture medium in an amount larger than a wild-type or parental strain, and preferably means that the microorganism is able to produce and secrete L-cysteine into a medium in an amount of at least 0.5 g/L, more preferably at least 1.0 g/L.

The phrase "a bacterium belonging to the genus *Escherichia"* means that the bacterium is classified as the genus *Escherichia* according to the classification known to a person skilled in the art of microbiology. A microorganism belonging to the genus *Escherichia* as used in the present invention includes, but is not limited to *Escherichia coli* (*E. coli*). *E. coli* is one of the most preferred bacterium of the present invention.

The phrase "modified to enhance expression of gene(s)" means that the expression amount of the gene(s) is greater than that of a non-modified strain, for example, a wild-type strain. For example, increasing the number of gene(s) per cell, increasing the number of molecules encoded by the gene(s) per cell, or increasing the gene expression level, and so forth, are encompassed. The copy number of the expressed gene is measured, for example, by restriction of chromosomal DNA followed by Southern blotting using a probe constructed based on the gene sequence, fluorescence *in situ* hybridization (FISH) and the like. The level of gene expression may be measured by various methods known in the art, including Northern blotting, quantitative RT-PCR, and the like. Furthermore, a wild-type strain, such as *Escherichia coli* K-12, may serve as a control. As a result of the enhancement of expression of the genes(s), L-cysteine secretion and accumulation in the medium is increased.

Enhancing the expression of the *cysPTWAM* cluster genes in a bacterial cell is achieved by increasing the copy number of the *cysPTWAM* cluster genes or modifying the cluster's expression control sequence so that the expression of the genes is enhanced.

The *cysPTWAM* cluster genes used in the present invention include those derived from bacteria belonging to the genus *Escherichia,* as well as those derived from other bacteria, such as *Salmonella.* Genes derived from bacteria belonging to the genus *Escherichia* are preferred.

Sequences of the *cysPTWAM* cluster genes from *Escherichia coli* have been reported as follows (Blattner, F.R. et al, Science, 277 (5331), 1453-1474 (1997)):
*cysP* gene (SEQ ID NO: 1) - nucleotide numbers 2540532 to 2541548 in the sequence of GenBank accession NC_000913.1 (gi: 16130350),
*cysT (cysU)* gene (SEQ ID NO: 3) - nucleotide numbers 2539699 to 2540532 in the sequence of GenBank accession NC_000913.1 (gi: 16130349),
*cysW* gene (SEQ ID NO: 5) - nucleotide numbers 2538824 to 2539699 in the sequence of GenBank accession NC_000913.1 (gi: 16132224),
*cysA* gene (SEQ ID NO: 7) - nucleotide numbers 2537737 to 2538834 in the sequence of GenBank accession NC_000913.1 for (gi: 16130348),
*cysM* gene (SEQ ID NO: 9) - nucleotide numbers 2536692 to 2537603 in the sequence of GenBank accession NC_000913.1 (gi: 16130347).

The *cysPTWAM* cluster is located between *ychM* ORF b2420 locus and b2426 locus on the chromosome of *E. coli* strain K-12. Therefore, these genes can be obtained by PCR (polymerase chain reaction; refer to White, T.J. *et al., Trends Genet., 5,* 185 (1989)) utilizing primers based on the reported nucleotide sequences of the genes. Genes encoding the proteins of sulphate/thiosulphate transport system which are derived from other microorganisms can be obtained in a similar manner.

Examples of the genes of the *cysPTWAM* cluster derived from *Escherichia coli* include following DNAs:
- the *cysP* gene which encodes the protein (A) or (B):
   (A) a protein having the amino acid sequence shown in SEQ ID NO: 2; or
   (B) a protein variant of the amino acid sequence shown in SEQ ID NO: 2, which exhibits an activity of thiosulphate periplasmic binding protein;
- the *cysT* gene which encodes the protein (C) or (D):
   (C) a protein having the amino acid sequence shown in SEQ ID NO: 4; or
   (D) a protein variant of the amino acid sequence shown in SEQ ID NO: 4, which exhibits an activity of sulphate/thiosulphate transport system permease when combined with proteins (E) or (F), and (G) or (H);
- the *cysW* gene which encodes the protein (E) or (F):
   (E) a protein having the amino acid sequence shown in SEQ ID NO: 6; or
   (F) a protein variant of the amino acid sequence shown in SEQ ID NO: 6, which exhibits an activity of sulphate/thiosulphate transport system permease when combined with proteins (C) or (D), and (G) or (H);
- the *cysA* gene which encodes the protein (G) or (H):
   (G) a protein having the amino acid sequence shown in SEQ ID NO: 8; or
   (H) a protein variant of the amino acid sequence shown in SEQ ID NO: 8, which is a ATP-binding component of sulphate/thiosulphate permease and exhibits an activity of the sulphate/thiosulphate transport system permease when combined with proteins (C) or (D), and (E) or (F);
- the *cysM* gene which encodes the protein (I) or (J):
   (I) a protein having the amino acid sequence shown in SEQ ID NO: 10; or
   (J) a protein variant of the amino acid sequence shown in SEQ ID NO: 10, which exhibits an activity of O-acetylserine(thiol)-lyase-B.

The phrase "an activity of sulphate/thiosulphate transport system permease" means an activity of a protein which transports thiosulphate into the cell from the outer medium. The phrase "an activity of O-acetylserine(thiol)-lyase-B" means an activity which catalyzes the reaction between O-acetyl-L-serine and thiosulphate, yielding S-sulfocysteine. The presence of these activities may be determined by, for example, complementation experiments using bacteria having mutations in the corresponding genes.

The DNA encoding proteins of the present invention includes a DNA encoding protein variants, possibly having deletions, substitutions, insertions or additions of one or several amino acids in one or more positions in the proteins (A), (C), (E), (G) or (I), as long as such changes do not result in loss of the protein's activity. The number of "several" amino acids differs depending on the position of amino acid residues in the three-dimensional structure of the protein and the type of the amino acids. However, it preferably means between 2 to 30, more preferably between 2 to 20, and most preferably between 2 to 10 for a protein having approximately 300 amino acid residues. This is because some amino acids have high homology to one another and the differences between the amino acid sequences does not greatly affect the three dimensional structure of the protein and its activity. Therefore, the protein (B), (D), (F), (H) or (J) may be one which has homology of not less than 30 to 50 %, preferably 50 to 70 %, more preferably 70 to 90 %, more preferably not less than 90%, and most preferably not less than 95% with respect to the entire amino acid sequence of the protein (A), (C), (E), (G) or (I), respectively, and which has the activity of the respective protein.

To evaluate the degree of protein or DNA homology, known calculation methods can be used, such as BLAST search, FASTA search and CrustalW.

BLAST (Basic Local Alignment Search Tool) is the heuristic search algorithm employed by the programs blastp, blastn, blastx, megablast, tblastn, and tblastx; these programs ascribe significance to their findings using the statistical methods of Karlin, Samuel and Stephen F. Altschul ("Methods for assessing the statistical significance of molecular sequence features by using general scoring schemes". Proc. Natl. Acad. Sci. USA, 1990, 87:2264-68; "Applications and statistics for multiple high-scoring segments in molecular sequences". Proc. Natl. Acad. Sci. USA, 1993, 90:5873-7). FASTA search method described by W. R. Pearson ("Rapid and Sensitive Sequence Comparison with FASTP and FASTA", Methods in Enzymology, 1990 183:63- 98). ClustalW method described by Thompson J.D., Higgins D.G. and Gibson T.J. ("CLUSTAL W: improving the sensitivity of progressive multiple sequence alignment through sequence weighting, position-specific gap penalties and weight matrix choice", Nucleic Acids Res. 1994, 22:4673-4680).

Changes to the protein defined in (A), (C), (E), (G) or (I) such as those described above are typically conservative changes so as to maintain the activity of each protein. Substitution changes include those in which at least one residue in the amino acid sequence has been removed and a different residue inserted in its place. Examples of amino acids which may be substituted for an original amino acid in the above protein and which are regarded as conservative substitutions include: Ala substituted with ser or thr; arg substituted with gln, his, or lys; asn substituted with glu, gln, lys, his, asp; asp substituted with asn, glu, or gln; cys substituted with ser or ala; gln substituted with asn, glu, lys, his, asp, or arg; glu substituted with asn, gln, lys, or asp; gly substituted with pro; his substituted with asn, lys, gln, arg, tyr; ile substituted with leu, met, val, phe; leu substituted with ile, met, val, phe; lys substituted with asn, glu, gln, his, arg; met substituted with ile, leu, val, phe; phe substituted with trp, tyr, met, ile, or leu; ser substituted with thr, ala; thr substituted with ser or ala; trp substituted with phe, tyr; tyr substituted with his, phe, or trp; and val substituted with met, ile, leu.

The DNA encoding substantially the same proteins as the protein defined in (A), (C), (E), (G) or (I), such as a protein variant, may be obtained by, for example, modification of the nucleotide sequence encoding the protein defined in (A), (C), (E), (G) or (I) using site-directed mutagenesis so that one or more amino acid residue will be deleted, substituted, inserted or added. This modified DNA can be obtained by conventional methods of treatment with reagents under conditions which typically generate mutations. These treatments include treating the DNA which encodes proteins of present invention with hydroxylamine, or treating the bacterium harboring the DNA with UV irradiation or a reagent such as N-methyl-N'-nitro-N-nitrosoguanidine or nitrous acid.

The DNA of the *cysPTWAM* cluster genes include variants derived from different strains and variants of bacteria belonging to the genus *Escherichia* by virtue of natural diversity.

DNA encoding such variants can be obtained by isolating DNA which hybridizes to the *cysP, cysT, cysW, cysA* or *cysM* gene or a part thereof under stringent conditions, and which encodes the protein having an inherent activity of the protein encoded by each of the genes. The term "stringent conditions" may include conditions under which a so-called specific hybrid is formed, and a non-specific hybrid is not formed. For example, stringent conditions include conditions under which DNAs having high homology, for instance DNAs having homology not less than 70%, preferably not less than 80 %, more preferably not less than 90%, most preferably not less than 95% to each other, are able to hybridize. Alternatively, stringent conditions may include typical washing conditions for Southern hybridization, e.g., 60°C, 1 x SSC, 0.1% SDS, preferably 0.1 x SSC, 0.1% SDS. Duration of the washing procedure depends on the type of membrane used for blotting and, as a rule, is recommended by manufacturer. For example, recommended duration of washing the Hybond™ N+ nylon membrane (Amersham) under stringent conditions is 15 minutes. As a probe for the DNA that encodes variants and hybridizes with the *cysP, cysT, cysW, cysA* or *cysM* gene, a partial sequence of the nucleotide sequence of SEQ ID NO: 1, 3, 5, 7 or 9 can also be used. Such a probe may be prepared by PCR using oligonucleotides based on the nucleotide sequence of SEQ ID NO: 1, 3, 5, 7 or 9 as primers, and a DNA fragment containing the nucleotide sequence of SEQ ID NO: 1, 3, 5, 7 or 9 as a template. When a DNA fragment of about 300 bp in length is used as the probe, the washing conditions for the hybridization can be, for example, 50°C, 2 x SSC, and 0.1% SDS.

Methods for enhancing gene expression include increasing the gene copy number. Introducing a gene into a vector that is able to function in a bacterium belonging to the genus *Escherichia* will increase the copy number of the gene. Multi-copy vectors can be preferably used, and include pBR322, pUC19, pBluescript KS⁺, pACYC177, pACYC184, pAYC32, pMW119, pET22b and the like. Enhancing gene expression can be achieved by introducing multiple copies of the gene into the bacterial chromosome via, for example, homologous recombination methods and the like.

Transforming a bacterium with a DNA harboring a gene encoding a protein means introduction of the DNA into the bacterium, for example, by conventional methods, to increase expression of the gene in the bacterium.

Furthermore, enhancing gene expression can be achieved by placing the DNA of the present invention under the control of a more potent promoter rather than the native promoter. The term "native promoter" means a DNA region which is present in a wild-type organism, located upstream of the open reading frame (ORF) of the gene, and promotes expression of the gene. Translational coupling of genes in the *cysPTWA* cluster indicates that these genes are expressed as a single transcription unit from a promoter just upstream of *cysP* gene (Hryniewicz, M.M., Kredich, N.M., J. Bacteriol., 173(18), 5876-86 (1991)). *cysM* gene is separated from *cysA* gene by only 174 bp in the chromosome of *E*. *coli* and may also be part of this operon, which is transcribed counterclockwise on the chromosome (*Escherichia coli* and *Salmonella,* Second Edition, Editor in Chief: F.C.Neidhardt, ASM Press, Washington D.C., 1996). Promoter strength is defined as the frequency of acts of RNA synthesis initiation. Methods for evaluating the strength of a promoter are described by, for example, Deuschle U., Kammerer W., Gentz R., Bujard H. (Promoters in Escherichia coli: a hierarchy of in vivo strength indicates alternate structures. EMBO J. 1986, 5, 2987-2994).

Enhancing translation can also be achieved by introducing into the DNA of the present invention a more efficient Shine-Dalgarno sequence (SD sequence). The SD sequence is a region upstream of the start codon of mRNA which interacts with the 16S RNA of ribosome (Shine J. and Dalgarno L., Proc. Natl. Acad. Sci. USA, 1974, 71, 4, 1342-6). The term "native SD sequence" means the SD sequence present in the wild-type organism. An example of an efficient SD sequence includes the SD sequence of the ϕ*10* gene from phage T7 (Olins P.O. et al, Gene, 1988, 73, 227-235).

Use of potent promoters can be combined with multiplication of gene copies. It is also possible to increase the copy number of genes of *cysPTWAM* cluster by combining the integration of one or several genes of the cluster with introduction of one of several genes into a multi-copy vector.

Methods for preparation of chromosomal DNA, hybridization, PCR, preparation of plasmid DNA, digestion and ligation of DNA, transformation, selection of an oligonucleotide as a primer and the like include typical methods well known to one of ordinary skill in the art. Such methods are described in Sambrook, J., and Russell D., "Molecular Cloning A Laboratory Manual, Third Edition", Cold Spring Harbor Laboratory Press (2001) and the like.

The bacterium of the present invention can be obtained by introduction of the aforementioned DNAs into a bacterium which inherently has the ability to produce L-cysteine. Alternatively, the bacterium of present invention can be obtained by imparting the ability to produce L-cysteine to the bacterium already harboring the DNAs.

The parent strain which is to be modified to have enhanced activity of the protein of the present invention may include an L-cysteine-producing bacterium belonging to the genus *Escherichia,* such as *E. coli* strain JM15 which is transformed with different *cysE* alleles encoding feedback-resistant serine acetyltransferases (US patent 6,218,168, Russian published patent application RU 2003121601); *E. coli* strain W3110 having overexpressed genes which encode a protein able to secrete toxic substances from cells (US patent 5,972,663); *E. coli* strains with low cysteine desulfhydrase activity (JP11155571A2); *E. coli* strain W3110 with increased activity of a positive transcriptional regulator for cysteine regulon coded by *cysB* gene (PCT application WO0127307A1) and the like.

It has been reported that the *ydeD* gene, which encodes a membrane protein not involved in the biosynthetic pathway of any L-amino acid, can enhance production of L-cysteine when additional copies of the gene are introduced into cells of the respective producing strain (US patent 5,972,663). Therefore, an embodiment of the present invention includes the L-cysteine-producing bacterium which is further modified to have enhanced expression of the *ydeD* open reading frame.

The bacterium of the present invention may also have enhanced expression of L-cysteine biosynthesis genes. Such genes include the *cysE* gene, which encodes feed-back resistant serine acetyltransferase (US patent 6,218,168), the *serA* gene, which encodes feed-back resistant phosphoglycerate dehydrogenase (US patent 6,180,373), and the like.

Proteins encoded by the *ydeD, cysE* or *serA* genes may be variants in the same manner as described for the *cysP, cysT, cysW, cysA* or *cysM* gene products. The method of present invention includes production of L-cysteine comprising the steps of cultivating the bacterium of the present invention in a culture medium, allowing the L-cysteine to be produced by the bacterium and secreted, and collecting the L-cysteine from the culture medium.

In the present invention, the cultivation, collection and purification of L-cysteine from the medium and the like may be performed by conventional fermentation and purification methods typically used for production and isolation of an amino acid using a microorganism.

The medium used for culture may be either a synthetic medium or a natural medium, so long as the medium includes a carbon source, a nitrogen source, a sulphur source and minerals and, if necessary, appropriate amounts of nutrients which the chosen microorganism requires for growth.

The carbon source may include various carbohydrates such as glucose and sucrose, and various organic acids. Depending on the mode of assimilation of the chosen microorganism, alcohol, including ethanol and glycerol, may be used.

As the nitrogen source, various ammonium salts such as ammonia and ammonium salts, other nitrogen compounds such as amines, a natural nitrogen source such as peptone, soybean-hydrolysate and digested fermentative microorganism can be used.

Thiosulphates may be used as the sulphur source for the present invention.

Potassium monophosphate, sodium chloride, calcium chloride, magnesium salts, ferrous salts, manganese salts and the like may be used as minerals.

Some additional nutrients can be added to the medium if necessary. For instance, if the microorganism requires methionine for growth (methionine auxotrophy), a sufficient amount of methionine can be added to the medium for cultivation.

The cultivation is preferably performed under aerobic conditions such as by shaking, and/or stirring with aeration, at a temperature of 20 to 42 °C, preferably 34 to 40 °C. The pH of the culture is usually between 5 and 9, preferably between 6.5 and 7.2. The pH of the culture can be adjusted with ammonia, calcium carbonate, various acids, various bases, and buffers. Usually, a 1 to 5-day cultivation leads to the production, secretion, and accumulation of L-cysteine in the liquid medium.

After cultivation, solids such as cells can be removed from the liquid medium by centrifugation or membrane filtration, and the L-cysteine can be collected and purified by methods such as ion-exchange, concentration and/or crystallization.

### Examples

The present invention will be more concretely explained with reference to the following non-limiting Examples. In the Examples an amino acid is of L-configuration unless otherwise noted. Chromosomal DNA of *E*. *coli* strain MG1655 was used as a template in all PCRs.

### Example 1: Construction of the plasmid carrying ydeD gene, mutant cysE gene and mutant serA5 gene

It has been reported that the *ydeD* gene, which encodes a transmembrane protein, is useful for cysteine production (US patent 5, 972,663). Therefore, the *ydeD* gene was cloned by PCR using primers ydeD299F and ydeD299R (SEQ ID NOS: 19 and 20, respectively) and chromosomal DNA from *E. coli* strain MG1655.

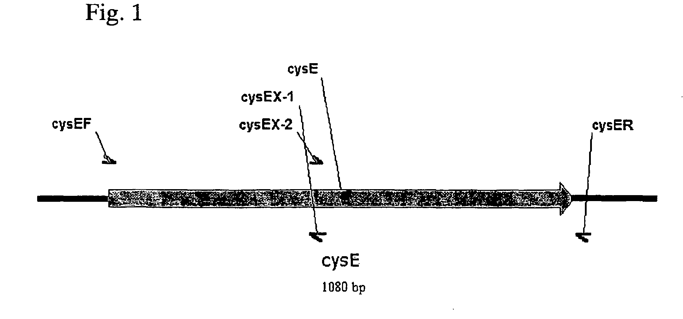

Then, mutant *cysEX* gene, which encodes serine acetyltransferase free from feedback inhibition by cysteine described in the US patent 6,218,168, was constructed by two successive PCR procedures for site-directed mutagenesis using primers cysEF and cysEX-1, cysEX-2 and cysER (SEQ ID NOS: 21 and 22,23 and 24, respectively) and chromosomal DNA from *E. coli* strain MG1655 as it shown on Figure 1. Two resulted PCR products were separated by electrophoresis and eluted from gel. In the second PCR these two DNA fragments were annealed and mutant *cysEX* gene was completed.

Also, the mutant *serA5* gene, which encodes the phosphoglycerate dehydrogenase free from feedback inhibition by serine described in the US patent 6,180,373, was cloned by PCR using primers serA5F and serA5R (SEQ ID NOS: 25 and 26, respectively) and chromosomal DNA from *E. coli* strain MG1655. Serine is the precursor of L-cysteine, so amplification of the mutant *serA5* gene is necessary to increase the amount of serine.

Finally, promoter P_{ompA} was cloned by PCR using primers depicted in SEQ ID No: 11 (primer PrOMPAF) and No. 12 (primer PrOMPAR). The primer PrOMPAF contains a restriction enzyme *Sal*I recognition site which has been introduced at the 5'-end thereof. A *Sal*I site was also introduced into forward primers for amplification of *ydeD* (SEQ ID NO: 19), *cysEX* (SEQ ID NO: 21) and *serA5* (SEQ ID NO: 25) genes. So, the *Sal*I site was used for assembling promoter P_{ompA} and each of genes. The primer PrOMPAR contains a restriction enzyme *Pae*I recognition site introduced at the 5'-end thereof. These restriction sites were introduced for further assembly of the genes and construction of a plasmid which is used for transformation.

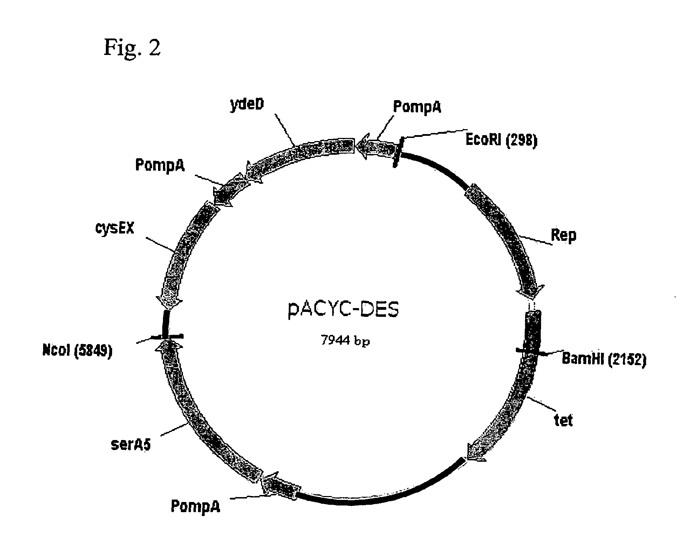

Then all three genes, each under promoter P_{ompA}, were cloned into vector pACYC184. Thus, the plasmid pACYC-DES was obtained (Figure 2).

### Example 2: Cloning of the cysPTWA genes from E. coli

The *cysPTWA* genes,which encode proteins of the sulphate/thiosulphate transport system, were cloned using the primers depicted in SEQ ID No: 13 (primer Mz025) and No. 14 (primer Mz026). The primer Mz025 is identical to a sequence starting 315 bp upstream of the start codon of the *cysP* gene, and having a restriction enzyme *PaeI* recognition site introduced at the 5'-end thereof. The primer Mz026 is a sequence complementary to a sequence starting 13 bp downstream of the termination codon of *cysA* gene and having a restriction enzyme *Sal*I recognition site introduced at the 5'-end thereof. The resulting PCR fragment, which contains the *cysPTWA* cluster under its own promoter, was treated with *Pae*I and *Sal*I restrictases and inserted into vector pMW119, which had been previously treated with the same enzymes. Thus plasmid pMW-PTWA was obtained.

### Example 3. Cloning of the cysM gene from E. coli

The *cysM* gene encoding O-acetylserine(thiol)-lyase-B was cloned by PCR using the primers depicted in SEQ ID No: 15 (primer cysMF) and No. 16 (primer cysMR). The primer cysMF contains a restriction enzyme *Sal*I recognition site which has been introduced at the 5'-end thereof. The primer cysMR contains a restriction enzyme *Xba*I recognition site which has been introduced at the 5'-end thereof.

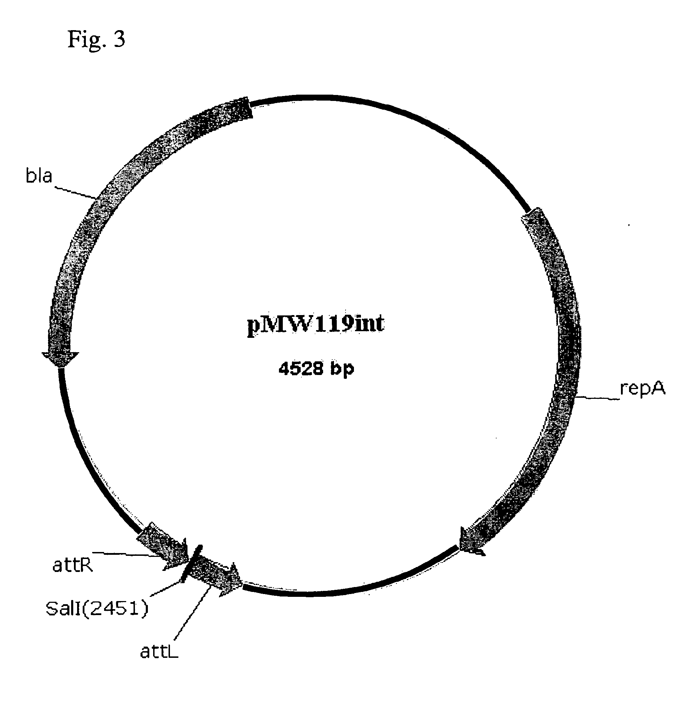

Promoter P_{cysK} was obtained by PCR using primers depicted in SEQ ID No: 17 (primer PcyskF) and No. 18 (primer PcysKR). The primer PcysKF is identical to a sequence starting 6 bp upstream of the start codon of *cysK* gene, and a restriction enzyme *Sal*I recognition site has been introduced at the 5'-end thereof. The primer PcysKR is complementary to a sequence starting 301 bp upstream of the start codon of *cysK* gene, and a restriction enzyme *PaeI* recognition site has been introduced at the 5'-end thereof. Then, the two resulting PCR products were assembled by treatment with restrictase *SalI* followed by ligation, and introduced into the integrative vector pMW119int. Vector pMW119int (Figure 3) was constructed from the commercially available vector pMW119 by insertion of attR and attL sites, which are necessary for further Mu-integration into *SalI* site of pMW119. Thus plasmid pMW-P_{cysK}-cysM was obtained.

### Example 4. Construction of the E. coli strain with an altered system for L-cysteine degradation

The *tnaA* and *metC* genes encode proteins of the main cysteine degradation pathway (Japanese publised patent application JP2003169668). Therefore, an *E. coli* strain MG1655 with an altered L-cysteine degradation (tnaA⁻, metC⁻) system was constructed as follows.

Initially, a mutation in the *metC* gene was induced by N-methyl-N'-nitro-N-nitrosoguanidine (NTG) treatment followed by multiple procedures of ampicillin enrichment. The mutant, which was able to grow on cystathionine, but not on homocysteine, was selected. Then, the disrupted *tnaA* gene from the strain CGSC7152 (tnaA300::Tn10(Tc^{R}), *E. coli* Genetic Stock Center, USA) was transferred into the resulting metC⁻ strain by the standard procedure of P1 transduction (Sambrook et al, "Molecular Cloning A Laboratory Manual, Second Edition", Cold Spring Harbor Laboratory Press (1989)).

Thus the *E. coli* strain MT was obtained.

### Example 5: Effect of enhanced expression of cysPTWAM cluster on L-cysteine production.

The *E. coli* strain MT was used as a parental strain to evaluate the effect of enhanced expression of the *cysPTWAM* cluster on L-cysteine production.

Initially, the *cysM* gene was integrated into the chromosome of strain MT using the plasmid pMW-P_{cysK}-cysM by the standard procedure of Mu-integration.

Then, plasmids pACYC-DES and pMW-PTWA were subsequently introduced into the resulting transductant MTintCYSM, giving strains MTintCYSM/pACYC-DES and MTintCYSM/pACYC-DES/pMW-PTWA.

Both strains MTintCYSM/pACYC-DES and MTintCYSM/pACYC-DES/pMW-PTWA were cultivated overnight with shaking at 34 °C in 2 ml of nutrient broth which had been supplemented with 50 mg/l of ampicillin and 20 µg/ml of tetracycline. 0.2 ml of the resulting cultures were inoculated into 2 ml of a fermentation medium containing tetracycline (20 mg/l) and ampicillin (50 mg/l) in 20 x 200 mm test tubes, and cultivated at 34 °C for 42 hours with a rotary shaker at 250 rpm. The composition of the fermentation medium was 15.0 g/l of (NH₄)₂SO₄, 1.5 g/l of KH₂PO₄, 1.0 g/l of MgSO₄, 20.0 g/l of CaCO₃, 0.1 mg/l of thiamine, 1% of Luria broth (LB medium), 4% of glucose, 300 mg/l of L-methionine and varied concentrations of Na₂S₂O₃.

After the cultivation, the amount of L-cysteine which had accumulated in the medium was determined by the method described by Gaitonde, M.K. (Biochem. J., 104:2, 627-33 (1967)). Data are presented in Table 1.

**Table 1**

| Strains | Thiosulphate concentration, g/l | Amount of L-cysteine, g/l |
|---|---|---|
| MTintCYSM/pACYC-DES | 2.5 | 3.1 |
| | 5.0 | 3.4 |
| | 7.0 | 3.8 |
| | 10.0 | 2.8 |
| MTintCYSM/pACYC-DES/pMW-PTWA | 2.5 | 4.1 |
| | 5.0 | 5.6 |
| | 7.0 | 6.2 |
| | 10.0 | 5.5 |

For mini-jar fermentation, one loop of each strain MTintCYSM/pACYC-DES and MTintCYSM/pACYC-DES/pMW-PTWA grown on L-agar was transferred to L-broth and cultivated at 34 °C with rotation (140 rpm) to reach optical density of culture OD₅₄₀ ≈ 2.0. Then 25 ml of seed culture was added to 250 ml of medium for fermentation and cultivated at 34 °C with rotation (1500 rpm) for 48 hours.

The composition of the fermentation medium for jar-fermenter (g/l):

| | | |
|---|---|---|
| Tryptone | | 2.0 |
| Yeast extract | | 1.0 |
| (NH4)₂SO₄ | | 5.0 |
| KH₂PO₄ | | 1.5 |
| NaCI | | 0.5 |
| MgSO₄•7H₂O | 0.3 | |
| CaCl₂•2H₂O | | 0.015 |
| FeSO₄•7H₂O | | 0.075 |
| Sodium citrate•2H₂O | 1.0 | |
| Glucose | | 100.0 |
| Methionine | | 0.45 |

The medium was supplemented with thiosulfate after 6 hours of fermentation at a velocity of 0.4 g/l*h.

After the cultivation, the amount of L-cysteine which had accumulated in the medium was determined as described above. Data are presented in the Table 2.

**Table 2**

| Strains | Amount of L-cysteine, g/l |
|---|---|
| MTintCYSM/pACYC-DES | 3.9 |
| MTintCYSM/pACYC-DES/pMW-PTWA | 6.6 |

As seen in Tables 1 and 2, enhanced expression of genes from *cysPTWAM* cluster improved cysteine productivity of the MT strain.

## Claims

1. An L-cysteine-producing bacterium belonging to the genus *Escherichia,* wherein the bacterium has been modified so that the expression amount of *cysPTWAM* cluster genes is greater than that of a strain before modification by increasing the copy number of the *cysPTWAM* cluster genes or modifying an expression control sequence of said genes.

2. The bacterium according to claim 1, wherein the copy number is increased by transforming the bacterium with a multi-copy vector harboring *cysPTWAM* cluster genes.

3. The bacterium according to claim 1, wherein the native promoter of said cluster is replaced with a promoter, which is more potent than the native promoter.

4. The bacterium according to claim 1, wherein the copy number is increased by integrating additional copies of said *cysPTWAM* cluster genes into the chromosome of the bacterium.

5. The bacterium according to claim 1, wherein said *cysPTWAM* cluster genes are derived from a bacterium belonging to the genus *Escherichia.*

6. The bacterium according to claim 5, wherein the bacterium is further modified so that the expression amount of the *ydeD* open reading frame is greater than that of a strain before modification.

7. A method for producing L-cysteine which comprises cultivating the bacterium according to claim 1 in a culture medium containing thiosulphate, and collecting L-cysteine from the culture medium.

8. The method according to claim 7, wherein the expression amount of L-cysteine biosynthesis genes in the bacterium is greater than that of a strain before modification.

## Patentansprüche

1. L-Cystein produzierendes Bakterium der Gattung Escherichia, wobei das Bakterium durch Erhöhen der Kopienzahl von cysPTWAM-Cluster-Genen oder durch Modifizieren einer Expressionskontrollsequenz der Gene so modifiziert worden ist, dass die exprimierte Menge der cysPTWAM-Cluster-Gene größer ist als diejenige eines Stammes vor der Modifizierung.

2. Bakterium nach Anspruch 1, wobei die Kopienzahl durch Transformieren des Bakteriums mit einem Mehrkopienvektor, der cysPTWAM-Cluster-Gene trägt, erhöht ist.

3. Bakterium nach Anspruch 1, wobei der native Promotor des Clusters durch einen Promotor ersetzt ist, der leistungsfähiger als der native Promotor ist.

4. Bakterium nach Anspruch 1, wobei die Kopienzahl durch Einführen zusätzlicher Kopien der cysPTWAM-Cluster-Gene in das Chromosom des Bakteriums erhöht ist.

5. Bakterium nach Anspruch 1, wobei die cysPTWAM-Cluster-Gene von einem Bakterium der Gattung Escherichia abgeleitet sind.

6. Bakterium nach Anspruch 5, wobei das Bakterium außerdem so modifiziert ist, dass die exprimierte Menge des offenen Leserahmens von ydeD größer ist als diejenige des Stammes vor der Modifizierung.

7. Verfahren zur Produktion von L-Cystein, welches das Kultivieren des Bakteriums nach Anspruch 1 in einer Kultur, die Thiosulfat enthält, und das Gewinnen von L-Cystein aus dem Kulturmedium umfasst.

8. Verfahren nach Anspruch 7, wobei die exprimierte Menge von L-Cystein-Biosynthesegenen in dem Bakterium größer ist als diejenige des Stammes vor der Modifizierung.

## Revendications

1. Bactérie productrice de L-cystéine appartenant au genre *Escherichia,* la bactérie ayant été modifiée de sorte à ce que l'intensité d'expression des gènes du groupe *cysPTWAM* soit supérieure à celle d'une souche avant modification par augmentation du nombre de copies des gènes du groupe *cysPTWAM* ou par modification d'une séquence de contrôle de l'expression desdits gènes.

2. Bactérie selon la revendication 1, dans laquelle le nombre de copies est augmenté par transformation de la bactérie à l'aide d'un vecteur multicopie hébergeant des gènes du groupe *cysPTWAM.*

3. Bactérie selon la revendication 1, dans laquelle le promoteur natif dudit groupe est remplacé par un promoteur qui est plus puissant que le promoteur natif.

4. Bactérie selon la revendication 1, dans laquelle le nombre de copies est augmenté par intégration de copies supplémentaires desdits gènes du groupe *cysPTWAM* dans le chromosome de la bactérie.

5. Bactérie selon la revendication 1, dans laquelle lesdits gènes du groupe *cysPTWAM* proviennent d'une bactérie appartenant au genre *Escherichia.*

6. Bactérie selon la revendication 5, la bactérie étant modifiée davantage de sorte à ce que l'intensité d'expression du cadre ouvert de lecture de *ydeD* soit supérieure à celle d'une souche avant modification.

7. Procédé de production de L-cystéine comprenant la culture de la bactérie selon la revendication 1 dans un milieu de culture contenant du thiosulfate, et la récolte de la L-cystéine du milieu de culture.

8. Procédé selon la revendication 7, dans lequel l'intensité d'expression des gènes de la biosynthèse de L-cystéine dans la bactérie est supérieure à ,celle d'une souche avant modification.


### SEQUENCE LISTING

<110> Ajinomoto Co., Inc.
<120> METHOD FOR PRODUCING L-CYSTEINE USING BACTERIA
   BELONGING TO THE GENUS ESCHERICHIA
<150> RU2003131993
   <151> 2003-11-03
<160> 26
<170> Patent In Ver. 2.1
<210> 1
   <211> 1017
   <212> DNA
   <213> Escherichia coli
<220>
   <221> CDS
   <222> (1)..(1017)
<400> 1
   atg gcc gtt aac tta ctg aaa aag aac tca ctc gcg ctg gtc gct tct      48
   Met Ala Val Asn Leu Leu Lys Lys Asn Ser Leu Ala Leu Val Ala Ser
    1               5                  10                  15
   ctg ctg ctg gcg ggc cat gta cag gca acg gaa ctg ctg aac agt tct      96
   Leu Leu Leu Ala Gly His Val Gln Ala Thr Glu Leu Leu Asn Ser Ser
               20                  25                  30
   tat gac gtc tcc cgc gag ctg ttt gcc gcc ctg aat ccg ccg ttt gag     144
   Tyr Asp Val Ser Arg Glu Leu Phe Ala Ala Leu Asn Pro Pro Phe Glu
           35                  40                  45
   caa caa tgg gca aaa gat aac ggc ggc gac aaa ctg acg ata aaa caa     192
   Gln Gln Trp Ala Lys Asp Asn Gly Gly Asp Lys Leu Thr Ile Lys Gln
       50                  55                  60
   tct cat gcc ggg tca tca aaa cag gcg ctg gcg att tta cag ggc tta     240
   Ser His Ala Gly Ser Ser Lys Gln Ala Leu Ala Ile Leu Gln Gly Leu
   65                  70                  75                  80
   aaa gcc gac gtt gtc act tat aac cag gtg acc gac gta caa atc ctg     288
   Lys Ala Asp Val Val Thr Tyr Asn Gln Val Thr Asp Val Gln Ile Leu
                   85                  90                  95
   cac gat aaa ggc aag ctg atc ccg gcc gac tgg cag tcg cgc ctg ccg     336
   His Asp Lys Gly Lys Leu Ile Pro Ala Asp Trp Gln Ser Arg Leu Pro
               100                 105                 110
   aat aat agc tcg ccg ttc tac tcc acc atg ggc ttc ctg gtg cgt aag     384
   Asn Asn Ser Ser Pro Phe Tyr Ser Thr Met Gly Phe Leu Val Arg Lys
           115                 120                 125
   ggt aac ccg aag aat atc cac gat tgg aac gac ctg gtg cgc tcc gac     432
   Gly Asn Pro Lys Asn Ile His Asp Trp Asn Asp Leu Val Arg Ser Asp
       130                 135                 140
   gtg aag ctg att ttc ccg aac ccg aaa acg tcg ggt aac gcg cgt tat     480
   Val Lys Leu Ile Phe Pro Asn Pro Lys Thr Ser Gly Asn Ala Arg Tyr
   145                 150                 155                 160
   acc tat ctg gcg gca tgg ggc gca gcg gat aaa gct gac ggt ggt gac     528
   Thr Tyr Leu Ala Ala Trp Gly Ala Ala Asp Lys Ala Asp Gly Gly Asp
                   165                 170                 175
   aaa ggc aaa acc gaa cag ttt atg acc cag ttc ctg aaa aac gtt gaa     576
   Lys Gly Lys Thr Glu Gln Phe Met Thr Gln Phe Leu Lys Asn Val Glu
               180                 185                 190
   gtg ttc gat act ggc ggt cgt ggc gcg acc acc act ttt gcc gag cgc     624
   Val Phe Asp Thr Gly Gly Arg Gly Ala Thr Thr Thr Phe Ala Glu Arg
           195                 200                 205
   ggc ctg ggc gat gtg ctg att agc ttc gaa tcg gaa gtg aac aac atc     672
   Gly Leu Gly Asp Val Leu Ile Ser Phe Glu Ser Glu Val Asn Asn Ile
       210                 215                 220
   cgt aaa cag tat gaa gcg cag ggc ttt gaa gtg gtg att ccg aaa acc     720
   Arg Lys Gln Tyr Glu Ala Gln Gly Phe Glu Val Val Ile Pro Lys Thr
   225                 230                 235                 240
   aac att ctg gcg gaa ttc ccg gtg gcg tgg gtt gat aaa aac gtg cag     768
   Asn Ile Leu Ala Glu Phe Pro Val Ala Trp Val Asp Lys Asn Val Gln
                   245                 250                 255
   gcc aac ggt acg gaa aaa gcc gcc aaa gcc tat ctg aac tgg ctc tat     816
   Ala Asn Gly Thr Glu Lys Ala Ala Lys Ala Tyr Leu Asn Trp Leu Tyr
               260                 265                 270
   agc ccg cag gcg caa acc atc atc acc gac tat tac tac cgc gtg aat     864
   Ser Pro Gln Ala Gln Thr Ile Ile Thr Asp Tyr Tyr Tyr Arg Val Asn
           275                 280                 285
   aac ccg gag gtg atg gac aaa ctg aaa gac aaa ttc ccg cag acc gag     912
   Asn Pro Glu Val Met Asp Lys Leu Lys Asp Lys Phe Pro Gln Thr Glu
       290                 295                 300
   ctg ttc cgc gtg gaa gac aaa ttt ggc tcc tgg ccg gaa gtg atg aaa     960
   Leu Phe Arg Val Glu Asp Lys Phe Gly Ser Trp Pro Glu Val Met Lys
   305                 310                 315                 320
   acc cac ttc acc agc ggc ggc gag tta gac aag ctg tta gcg gcg ggg     1008
   Thr His Phe Thr Ser Gly Gly Glu Leu Asp Lys Leu Leu Ala Ala Gly
                   325                 330                 335
   cgt aac tga                                                         1017
   Arg Asn
<210> 2
   <211> 338
   <212> PRT
   <213> Escherichia coli
<400> 2
   Met Ala Val Asn Leu Leu Lys Lys Asn Ser Leu Ala Leu Val Ala Ser
    1               5                  10                  15
   Leu Leu Leu Ala Gly His Val Gln Ala Thr Glu Leu Leu Asn Ser Ser
               20                  25                  30
   Tyr Asp Val Ser Arg Glu Leu Phe Ala Ala Leu Asn Pro Pro Phe Glu
           35                  40                  45
   Gln Gln Trp Ala Lys Asp Asn Gly Gly Asp Lys Leu Thr Ile Lys Gln
       50                  55                  60
   Ser His Ala Gly Ser Ser Lys Gln Ala Leu Ala Ile Leu Gln Gly Leu
   65                  70                  75                  80
   Lys Ala Asp Val Val Thr Tyr Asn Gln Val Thr Asp Val Gln Ile Leu
                   85                  90                  95
   His Asp Lys Gly Lys Leu Ile Pro Ala Asp Trp Gln Ser Arg Leu Pro
               100                 105                 110
   Asn Asn Ser Ser Pro Phe Tyr Ser Thr Met Gly Phe Leu Val Arg Lys
           115                 120                 125
   Gly Asn Pro Lys Asn Ile His Asp Trp Asn Asp Leu Val Arg Ser Asp
       130                 135                 140
   Val Lys Leu Ile Phe Pro Asn Pro Lys Thr Ser Gly Asn Ala Arg Tyr
   145                 150                 155                 160
   Thr Tyr Leu Ala Ala Trp Gly Ala Ala Asp Lys Ala Asp Gly Gly Asp
                   165                 170                 175
   Lys Gly Lys Thr Glu Gln Phe Met Thr Gln Phe Leu Lys Asn Val Glu
               180                 185                 190
   Val Phe Asp Thr Gly Gly Arg Gly Ala Thr Thr Thr Phe Ala Glu Arg
           195                 200                 205
   Gly Leu Gly Asp Val Leu Ile Ser Phe Glu Ser Glu Val Asn Asn Ile
       210                 215                 220
   Arg Lys Gln Tyr Glu Ala Gln Gly Phe Glu Val Val Ile Pro Lys Thr
   225                 230                 235                 240
   Asn Ile Leu Ala Glu Phe Pro Val Ala Trp Val Asp Lys Asn Val Gln
                   245                 250                 255
   Ala Asn Gly Thr Glu Lys Ala Ala Lys Ala Tyr Leu Asn Trp Leu Tyr
               260                 265                 270
   Ser Pro Gln Ala Gln Thr Ile Ile Thr Asp Tyr Tyr Tyr Arg Val Asn
           275                 280                 285
   Asn Pro Glu Val Met Asp Lys Leu Lys Asp Lys Phe Pro Gln Thr Glu
       290                 295                 300
   Leu Phe Arg Val Glu Asp Lys Phe Gly Ser Trp Pro Glu Val Met Lys
   305                 310                 315                 320
   Thr His Phe Thr Ser Gly Gly Glu Leu Asp Lys Leu Leu Ala Ala Gly
                   325                 330                 335
   Arg Asn
<210> 3
   <211> 834
   <212> DNA
   <213> Escherichia coli
<220>
   <221> CDS
   <222> (1)..(834)
<400> 3 275
<210> 4
   <211> 277
   <212> PRT
   <213> Escherichia coli
<400> 4
   Met Phe Ala Val Ser Ser Arg Arg Val Leu Pro Gly Phe Thr Leu Ser
    1               5                  10                  15
   Leu Gly Thr Ser Leu Leu Phe Val Cys Leu Ile Leu Leu Leu Pro Leu
               20                  25                  30
   Ser Ala Leu Val Met Gln Leu Ala Gln Met Ser Trp Ala Gln Tyr Trp
           35                  40                  45
   Glu Val Ile Thr Asn Pro Gln Val Val Ala Ala Tyr Lys Val Thr Leu
       50                  55                  60
   Leu Ser Ala Phe Val Ala Ser Ile Phe Asn Gly Val Phe Gly Leu Leu
   65                  70                  75                  80
   Met Ala Trp Ile Leu Thr Arg Tyr Arg Phe Pro Gly Arg Thr Leu Leu
                   85                  90                  95
   Asp Ala Leu Met Asp Leu Pro Phe Ala Leu Pro Thr Ala Val Ala Gly
               100                 105                 110
   Leu Thr Leu Ala Ser Leu Phe Ser Val Asn Gly Phe Tyr Gly Glu Trp
           115                 120                 125
   Leu Ala Lys Phe Asp Ile Lys Val Thr Tyr Thr Trp Leu Gly Ile Ala
       130                 135                 140
   Val Ala Met Ala Phe Thr Ser Ile Pro Phe Val Val Arg Thr Val Gln
   145                 150                 155                 160
   Pro Val Leu Glu Glu Leu Gly Pro Glu Tyr Glu Glu Ala Ala Glu Thr
                   165                 170                 175
   Leu Gly Ala Thr Arg Trp Gln Ser Phe Cys Lys Val Val Leu Pro Glu
               180                 185                 190
   Leu Ser Pro Ala Leu Val Ala Gly Val Ala Leu Ser Phe Thr Arg Ser
           195                 200                 205
   Leu Gly Glu Phe Gly Ala Val Ile Phe Ile Ala Gly Asn Ile Ala Trp
       210                 215                 220
   Lys Thr Glu Val Thr Ser Leu Met Ile Phe Val Arg Leu Gln Glu Phe
   225                 230                 235                 240
   Asp Tyr Pro Ala Ala Ser Ala Ile Ala Ser Val Ile Leu Ala Ala Ser
                   245                 250                 255
   Leu Leu Leu Leu Phe Ser Ile Asn Thr Leu Gln Ser Arg Phe Gly Arg
               260                 265                 270
   Arg Val Val Gly His
           275
<210> 5
   <211> 876
   <212> DNA
   <213> Escherichia coli
<220>
   <221> CDS
   <222> (1)..(876)
<400> 5
   atg gcg gaa gtt acc caa ttg aag cgt tat gac gcg cgc ccg att aac    48
   Met Ala Glu Val Thr Gln Leu Lys Arg Tyr Asp Ala Arg Pro Ile Asn
    1               5                  10                  15
    tgg ggc aaa tgg ttt ctg att ggc atc ggg atg ctg gtt tcg gcg ttc   96
   Trp Gly Lys Trp Phe Leu Ile Gly Ile Gly Met Leu Val Ser Ala Phe
               20                  25                  30
   atc ctg ctg gtg ccg atg att tac atc ttc gtg cag gca ttc agc aag   144
   Ile Leu Leu Val Pro Met Ile Tyr Ile Phe Val Gln Ala Phe Ser Lys
           35                  40                  45
   ggg ctg atg ccg gtt tta cag aat ctg gcc gat ccg gac atg ctg cac   192
   Gly Leu Met Pro Val Leu Gln Asn Leu Ala Asp Pro Asp Met Leu His
       50                  55                  60
   gcc atc tgg ctg acg gtg atg atc gcg ctg att gcc gta ccg gta aac   240
   Ala Ile Trp Leu Thr Val Met Ile Ala Leu Ile Ala Val Pro Val Asn
   65                  70                  75                  80
   ctg gtg ttc ggc att ctg ctg gcc tgg ctg gtg acg cgc ttt aac ttc   288
   Leu Val Phe Gly Ile Leu Leu Ala Trp Leu Val Thr Arg Phe Asn Phe
                   85                  90                  95
   cct gga cgc cag tta ctg ctg acg cta ctg gac att ccg ttt gcc gta   336
   Pro Gly Arg Gln Leu Leu Leu Thr Leu Leu Asp Ile Pro Phe Ala Val
               100                 105                 110
   tcg ccg gtg gtt gcc ggt ctg gtg tat ttg ctg ttc tac ggc tct aac   384
   Ser Pro Val Val Ala Gly Leu Val Tyr Leu Leu Phe Tyr Gly Ser Asn
           115                 120                 125
   ggc ccg ctc ggc ggt tgg ctc gac gag cat aac ctg caa att atg ttc   432
   Gly Pro Leu Gly Gly Trp Leu Asp Glu His Asn Leu Gln Ile Met Phe
       130                 135                 140
   tcc tgg ccg gga atg gtg ctg gtc acc atc ttc gtg acg tgt ccg ttt   480
   Ser Trp Pro Gly Met Val Leu Val Thr Ile Phe Val Thr Cys Pro Phe
   145                 150                 155                 160
   gtg gtg cgc gaa ctg gtg ccg gtg atg tta agc cag ggc agc cag gaa   528
   Val Val Arg Glu Leu Val Pro Val Met Leu Ser Gln Gly Ser Gln Glu
                   165                 170                 175
   gac gaa gcg gcg att ttg ctt ggc gcg tcc ggc tgg cag atg ttc cgt   576
   Asp Glu Ala Ala Ile Leu Leu Gly Ala Ser Gly Trp Gln Met Phe Arg
               180                 185                 190
   cgc gtc aca tta ccg aac atc cgc tgg gcg ctg ctt tat ggc gtg gtg   624
   Arg Val Thr Leu Pro Asn Ile Arg Trp Ala Leu Leu Tyr Gly Val Val
           195                 200                 205
   ttg acc aac gcc cgc gca att ggc gag ttt ggc gcg gtg tcg gtg gtt   672
   Leu Thr Asn Ala Arg Ala Ile Gly Glu Phe Gly Ala Val Ser Val Val
       210                 215                 220
   tcc ggc tcg att cgc ggc gaa acc ctg tcg ctg ccg tta cag att gaa   720
   Ser Gly Ser Ile Arg Gly Glu Thr Leu Ser Leu Pro Leu Gln Ile Glu
   225                 230                 235                 240
   ttg ctg gag cag gac tac aac acc gtc ggc tcc ttt acc gct gcg gcg   768
   Leu Leu Glu Gln Asp Tyr Asn Thr Val Gly Ser Phe Thr Ala Ala Ala
                   245                 250                 255
   ctg tta acg ctg atg gcg att atc acc ctg ttt tta aaa agt atg ttg   816
   Leu Leu Thr Leu Met Ala Ile Ile Thr Leu Phe Leu Lys Ser Met Leu
               260                 265                 270
   cag tgg cgc ctg gag aat cag gaa aaa cgc gca cag cag gag gaa cat    864
   Gln Trp Arg Leu Glu Asn Gln Glu Lys Arg Ala Gln Gln Glu Glu His
           275                 280                 285
   cat gag cat tga                                                    876
   His Glu His
       290
<210> 6
   <211> 291
   <212> PRT
   <213> Escherichia coli
<400> 6
   Met Ala Glu Val Thr Gln Leu Lys Arg Tyr Asp Ala Arg Pro Ile Asn
    1               5                  10                  15
   Trp Gly Lys Trp Phe Leu Ile Gly Ile Gly Met Leu Val Ser Ala Phe
               20                  25                  30
   Ile Leu Leu Val Pro Met Ile Tyr Ile Phe Val Gln Ala Phe Ser Lys
           35                  40                  45
   Gly Leu Met Pro Val Leu Gln Asn Leu Ala Asp Pro Asp Met Leu His
       50                  55                  60
   Ala Ile Trp Leu Thr Val Met Ile Ala Leu Ile Ala Val Pro Val Asn
   65                  70                  75                  80
   Leu Val Phe Gly Ile Leu Leu Ala Trp Leu Val Thr Arg Phe Asn Phe
                   85                  90                  95
   Pro Gly Arg Gln Leu Leu Leu Thr Leu Leu Asp Ile Pro Phe Ala Val
               100                 105                 110
   Ser Pro Val Val Ala Gly Leu Val Tyr Leu Leu Phe Tyr Gly Ser Asn
           115                 120                 125
   Gly Pro Leu Gly Gly Trp Leu Asp Glu His Asn Leu Gln Ile Met Phe
       130                 135                 140
   Ser Trp Pro Gly Met Val Leu Val Thr Ile Phe Val Thr Cys Pro Phe
   145                 150                 155                 160
   Val Val Arg Glu Leu Val Pro Val Met Leu Ser Gln Gly Ser Gln Glu
                   165                 170                 175
   Asp Glu Ala Ala Ile Leu Leu Gly Ala Ser Gly Trp Gln Met Phe Arg
               180                 185                 190
   Arg Val Thr Leu Pro Asn Ile Arg Trp Ala Leu Leu Tyr Gly Val Val
           195                 200                 205
   Leu Thr Asn Ala Arg Ala Ile Gly Glu Phe Gly Ala Val Ser Val Val
       210                 215                 220
   Ser Gly Ser Ile Arg Gly Glu Thr Leu Ser Leu Pro Leu Gln Ile Glu
   225                 230                 235                 240
   Leu Leu Glu Gln Asp Tyr Asn Thr Val Gly Ser Phe Thr Ala Ala Ala
                   245                 250                 255
   Leu Leu Thr Leu Met Ala Ile Ile Thr Leu Phe Leu Lys Ser Met Leu
               260                 265                 270
   Gln Trp Arg Leu Glu Asn Gln Glu Lys Arg Ala Gln Gln Glu Glu His
           275                 280                 285
   His Glu His
       290
<210> 7
   <211> 1098
   <212> DNA
   <213> Escherichia coli
<220>
   <221> CDS
   <222> (1)..(1098)
<400> 7
   atg agc att gag att gcc aat att aag aag tcg ttt ggt cgc acc cag    48
   Met Ser Ile Glu Ile Ala Asn Ile Lys Lys Ser Phe Gly Arg Thr Gln
    1               5                  10                  15
   gtg ctg aac gat atc tca ctg gat att cct tca ggt cag atg gtc gcg    96
   Val Leu Asn Asp Ile Ser Leu Asp Ile Pro Ser Gly Gln Met Val Ala
               20                  25                  30
   ttg ctg ggg ccg tcc ggt tcc ggg aaa acc acg ctg ctg cgc att atc    144
   Leu Leu Gly Pro Ser Gly Ser Gly Lys Thr Thr Leu Leu Arg Ile Ile
           35                  40                  45
   gcc ggg ctg gag cat caa acc agc ggg cat att cgc ttc cac ggc acc    192
   Ala Gly Leu Glu His Gln Thr Ser Gly His Ile Arg Phe His Gly Thr
       50                  55                  60
   gac gtg agc cgc ctg cac gca cgt gat cgt aaa gtc ggt ttc gtg ttc    240
   Asp Val Ser Arg Leu His Ala Arg Asp Arg Lys Val Gly Phe Val Phe
   65                  70                  75                  80
   cag cat tac gcg ctg ttc cgc cat atg acg gtg ttc gac aat atc gct    288
   Gln His Tyr Ala Leu Phe Arg His Met Thr Val Phe Asp Asn Ile Ala
                   85                  90                  95
   ttt ggc ctg acg gtg ctg ccg cgt cgc gag cgc ccg aat gcc gca gcc    336
   Phe Gly Leu Thr Val Leu Pro Arg Arg Glu Arg Pro Asn Ala Ala Ala
               100                 105                 110
   atc aaa gcg aaa gtg aca aaa ttg ctg gaa atg gtc cag ctt gcc cat    384
   Ile Lys Ala Lys Val Thr Lys Leu Leu Glu Met Val Gln Leu Ala His
           115                 120                 125
   ctg gcg gat cgt tat ccg gcg cag ctt tcc ggc ggc cag aaa cag cgc    432
   Leu Ala Asp Arg Tyr Pro Ala Gln Leu Ser Gly Gly Gln Lys Gln Arg
       130                 135                 140
   gtg gcg ctg gcg cgc gcg ctg gct gtg gaa ccg caa att ctg ctg ctt    480
   Val Ala Leu Ala Arg Ala Leu Ala Val Glu Pro Gln Ile Leu Leu Leu
   145                 150                 155                 160
   gat gaa ccg ttt ggc gcg ctg gat gcg cag gtg cgt aaa gag ctg cgt    528
   Asp Glu Pro Phe Gly Ala Leu Asp Ala Gln Val Arg Lys Glu Leu Arg
                   165                 170                 175
   cgc tgg ctg cgt caa ctc cat gaa gaa cta aaa ttc acc agc gtt ttt    576
   Arg Trp Leu Arg Gln Leu His Glu Glu Leu Lys Phe Thr Ser Val Phe
               180                 185                 190
   gtg acc cac gat cag gaa gaa gcg acc gaa gta gct gat cgt gta gtt    624
   Val Thr His Asp Gln Glu Glu Ala Thr Glu Val Ala Asp Arg Val Val
           195                 200                 205
   gtg atg agc cag ggc aat att gaa cag gct gac gcg ccg gat cag gta    672
   Val Met Ser Gln Gly Asn Ile Glu Gln Ala Asp Ala Pro Asp Gln Val
       210                 215                 220
   tgg cgc gaa ccg gcg acc cgt ttt gtg ctc gaa ttt atg ggc gaa gtg    720
   Trp Arg Glu Pro Ala Thr Arg Phe Val Leu Glu Phe Met Gly Glu Val
   225                 230                 235                 240
   aac cgc ctg cag gga acc att cgc ggc ggg cag ttc cat gtt ggc gcg    768
   Asn Arg Leu Gln Gly Thr Ile Arg Gly Gly Gln Phe His Val Gly Ala
                   245                 250                 255
   cat cgc tgg ccg ctg ggc tac aca cct gcg tat cag ggg ccg gtg gat    816
   His Arg Trp Pro Leu Gly Tyr Thr Pro Ala Tyr Gln Gly Pro Val Asp
               260                 265                 270
   ctc ttc ctg cgc cct tgg gaa gtg gat atc agc cgc cgt acc agc ctc    864
   Leu Phe Leu Arg Pro Trp Glu Val Asp Ile Ser Arg Arg Thr Ser Leu
           275                 280                 285
   gat tcg ccg ctg ccg gta cag gta ctg gaa gcc agc ccg aaa ggt cac    912
   Asp Ser Pro Leu Pro Val Gln Val Leu Glu Ala Ser Pro Lys Gly His
       290                 295                 300
   tac acc caa tta gtg gtg cag ccg ctg ggg tgg tac aac gaa ccg ctg    960
   Tyr Thr Gln Leu Val Val Gln Pro Leu Gly Trp Tyr Asn Glu Pro Leu
   305                 310                 315                 320
   acg gtc gtg atg cat ggc gac gat gcc ccg cag cgt ggc gag cgt tta    1008
   Thr Val Val Met His Gly Asp Asp Ala Pro Gln Arg Gly Glu Arg Leu
                   325                 330                 335
   ttc gtt ggt ctg caa cat gcg cgg ctg tat aac ggc gac gag cgt atc    1056
   Phe Val Gly Leu Gln His Ala Arg Leu Tyr Asn Gly Asp Glu Arg Ile
               340                 345                 350
   gaa acc cgc gat gag gaa ctt gct ctc gca caa agc gcc tga            1098
   Glu Thr Arg Asp Glu Glu Leu Ala Leu Ala Gln Ser Ala
           355                 360                 365
<210> 8
   <211> 365
   <212> PRT
   <213> Escherichia coli
<400> 8
   Met Ser Ile Glu Ile Ala Asn Ile Lys Lys Ser Phe Gly Arg Thr Gln
   1               5                   10                  15
   Val Leu Asn Asp Ile Ser Leu Asp Ile Pro Ser Gly Gln Met Val Ala
               20                  25                  30
   Leu Leu Gly Pro Ser Gly Ser Gly Lys Thr Thr Leu Leu Arg Ile Ile
           35                  40                  45
   Ala Gly Leu Glu His Gln Thr Ser Gly His Ile Arg Phe His Gly Thr
       50                  55                  60
   Asp Val Ser Arg Leu His Ala Arg Asp Arg Lys Val Gly Phe Val Phe
   65                  70                  75                  80
   Gln His Tyr Ala Leu Phe Arg His Met Thr Val Phe Asp Asn Ile Ala
                   85                  90                  95
   Phe Gly Leu Thr Val Leu Pro Arg Arg Glu Arg Pro Asn Ala Ala Ala
               100                 105                 110
   Ile Lys Ala Lys Val Thr Lys Leu Leu Glu Met Val Gln Leu Ala His
           115                 120                 125
   Leu Ala Asp Arg Tyr Pro Ala Gln Leu Ser Gly Gly Gln Lys Gln Arg
       130                 135                 140
   Val Ala Leu Ala Arg Ala Leu Ala Val Glu Pro Gln Ile Leu Leu Leu
   145                 150                 155                 160
   Asp Glu Pro Phe Gly Ala Leu Asp Ala Gln Val Arg Lys Glu Leu Arg
                   165                 170                 175
   Arg Trp Leu Arg Gln Leu His Glu Glu Leu Lys Phe Thr Ser Val Phe
               180                 185                 190
   Val Thr His Asp Gln Glu Glu Ala Thr Glu Val Ala Asp Arg Val Val
           195                 200                 205
   Val Met Ser Gln Gly Asn Ile Glu Gln Ala Asp Ala Pro Asp Gln Val
       210                 215                 220
   Trp Arg Glu Pro Ala Thr Arg Phe Val Leu Glu Phe Met Gly Glu Val
   225                 230                 235                 240
   Asn Arg Leu Gln Gly Thr Ile Arg Gly Gly Gln Phe His Val Gly Ala
                   245                 250                 255
   His Arg Trp Pro Leu Gly Tyr Thr Pro Ala Tyr Gln Gly Pro Val Asp
               260                 265                 270
   Leu Phe Leu Arg Pro Trp Glu Val Asp Ile Ser Arg Arg Thr Ser Leu
           275                 280                 285
   Asp Ser Pro Leu Pro Val Gln Val Leu Glu Ala Ser Pro Lys Gly His
       290                 295                 300
   Tyr Thr Gln Leu Val Val Gln Pro Leu Gly Trp Tyr Asn Glu Pro Leu
   305                 310                 315                 320
   Thr Val Val Met His Gly Asp Asp Ala Pro Gln Arg Gly Glu Arg Leu
                   325                 330                 335
   Phe Val Gly Leu Gln His Ala Arg Leu Tyr Asn Gly Asp Glu Arg Ile
               340                 345                 350
   Glu Thr Arg Asp Glu Glu Leu Ala Leu Ala Gln Ser Ala
           355                 360                 365
<210> 9
   <211> 912
   <212> DNA
   <213> Escherichia coli
<220>
   <221> CDS
   <222> (1)..(912)
<400> 9
   gtg agt aca tta gaa caa aca ata ggc aat acg cct ctg gtg aag ttg    48
   Val Ser Thr Leu Glu Gln Thr Ile Gly Asn Thr Pro Leu Val Lys Leu
   1               5                   10                  15
   cag cga atg ggg ccg gat aac ggc agt gaa gtg tgg tta aaa ctg gaa    96
   Gln Arg Met Gly Pro Asp Asn Gly Ser Glu Val Trp Leu Lys Leu Glu
               20                  25                  30
   ggc aat aac ccg gca ggt tcg gtg aaa gat cgt gcg gca ctt tcg atg   144
   Gly Asn Asn Pro Ala Gly Ser Val Lys Asp Arg Ala Ala Leu Ser Met
           35                  40                  45
   atc gtc gag gcg gaa aag cgc ggg gaa att aaa ccg ggt gat gtc tta   192
   Ile Val Glu Ala Glu Lys Arg Gly Glu Ile Lys Pro Gly Asp Val Leu
       50                  55                  60
    atc gaa gcc acc agt ggt aac acc ggc att gcg ctg gca atg att gcc    240
    Ile Glu Ala Thr Ser Gly Asn Thr Gly Ile Ala Leu Ala Met Ile Ala
   65                  70                  75                  80
   gcg ctg aaa ggc tat cgc atg aaa ttg ctg atg ccc gac aac atg agc    288
   Ala Leu Lys Gly Tyr Arg Met Lys Leu Leu Met Pro Asp Asn Met Ser
                   85                  90                  95
   cag gaa cgc cgt gcg gcg atg cgt gct tat ggt gcg gaa ctg att ctt    336
   Gln Glu Arg Arg Ala Ala Met Arg Ala Tyr Gly Ala Glu Leu Ile Leu
               100                 105                 110
   gtc acc aaa gag cag ggc atg gaa ggt gcg cgc gat ctg gcg ctg gag    384
   Val Thr Lys Glu Gln Gly Met Glu Gly Ala Arg Asp Leu Ala Leu Glu
           115                 120                 125
   atg gcg aat cgt ggc gaa gga aag ctg ctc gat cag ttc aat aat ccc    432
   Met Ala Asn Arg Gly Glu Gly Lys Leu Leu Asp Gln Phe Asn Asn Pro
       130                 135                 140
   gat aac cct tat gcg cat tac acc acc act ggg ccg gaa atc tgg cag    480
   Asp Asn Pro Tyr Ala His Tyr Thr Thr Thr Gly Pro Glu Ile Trp Gln
   145                 150                 155                 160
   caa acc ggc ggg cgc atc act cat ttt gtc tcc agc atg ggg acg acc    528
   Gln Thr Gly Gly Arg Ile Thr His Phe Val Ser Ser Met Gly Thr Thr
                   165                 170                 175
   ggc act atc acc ggc gtc tca cgc ttt atg cgc gaa caa tcc aaa ccg    576
   Gly Thr Ile Thr Gly Val Ser Arg Phe Met Arg Glu Gln Ser Lys Pro
               180                 185                 190
   gtg acc att gtc ggc ctg caa ccg gaa gag ggc agc agc att ccc ggc    624
   Val Thr Ile Val Gly Leu Gln Pro Glu Glu Gly Ser Ser Ile Pro Gly
           195                 200                 205
   att cgc cgc tgg cct acg gaa tat ctg ccg ggg att ttc aac gct tct    672
   Ile Arg Arg Trp Pro Thr Glu Tyr Leu Pro Gly Ile Phe Asn Ala Ser
       210                 215                 220
   ctg gtg gat gag gtg ctg gat att cat cag cgc gat gcg gaa aac acc    720
   Leu Val Asp Glu Val Leu Asp Ile His Gln Arg Asp Ala Glu Asn Thr
   225                 230                 235                 240
   atg cgc gaa ctg gcg gtg cgg gaa gga ata ttc tgt ggc gtc agc tcc    768
   Met Arg Glu Leu Ala Val Arg Glu Gly Ile Phe Cys Gly Val Ser Ser
                   245                 250                 255
   ggc ggc gcg gtt gcc gga gca ctg cgg gtg gca aaa gct aac cct gac    816
   Gly Gly Ala Val Ala Gly Ala Leu Arg Val Ala Lys Ala Asn Pro Asp
               260                 265                 270
   gcg gtg gtg gtg gcg atc atc tgc gat cgt ggc gat cgc tac ctt tct    864
   Ala Val Val Val Ala Ile Ile Cys Asp Arg Gly Asp Arg Tyr Leu Ser
           275                 280                 285
   acc ggg gtg ttt ggg gaa gag cat ttt agc cag ggg gcg ggg att taa    912
   Thr Gly Val Phe Gly Glu Glu His Phe Ser Gln Gly Ala Gly Ile
       290                 295                 300
<210> 10
   <211> 303
   <212> PRT
   <213> Escherichia coli
<400> 10
   Val Ser Thr Leu Glu Gln Thr Ile Gly Asn Thr Pro Leu Val Lys Leu
   1               5                   10                  15
   Gln Arg Met Gly Pro Asp Asn Gly Ser Glu Val Trp Leu Lys Leu Glu
               20                  25                  30
   Gly Asn Asn Pro Ala Gly Ser Val Lys Asp Arg Ala Ala Leu Ser Met
           35                  40                  45
   Ile Val Glu Ala Glu Lys Arg Gly Glu Ile Lys Pro Gly Asp Val Leu
       50                  55                  60
   Ile Glu Ala Thr Ser Gly Asn Thr Gly Ile Ala Leu Ala Met Ile Ala
   65                  70                  75                  80
   Ala Leu Lys Gly Tyr Arg Met Lys Leu Leu Met Pro Asp Asn Met Ser
                   85                  90                  95
   Gln Glu Arg Arg Ala Ala Met Arg Ala Tyr Gly Ala Glu Leu Ile Leu
               100                 105                 110
   Val Thr Lys Glu Gln Gly Met Glu Gly Ala Arg Asp Leu Ala Leu Glu
           115                 120                 125
   Met Ala Asn Arg Gly Glu Gly Lys Leu Leu Asp Gln Phe Asn Asn Pro
       130                 135                 140
   Asp Asn Pro Tyr Ala His Tyr Thr Thr Thr Gly Pro Glu Ile Trp Gln
   145                 150                 155                 160
   Gln Thr Gly Gly Arg Ile Thr His Phe Val Ser Ser Met Gly Thr Thr
                   165                 170                 175
   Gly Thr Ile Thr Gly Val Ser Arg Phe Met Arg Glu Gln Ser Lys Pro
               180                 185                 190
   Val Thr Ile Val Gly Leu Gln Pro Glu Glu Gly Ser Ser Ile Pro Gly
           195                 200                 205
   Ile Arg Arg Trp Pro Thr Glu Tyr Leu Pro Gly Ile Phe Asn Ala Ser
       210                 215                 220
   Leu Val Asp Glu Val Leu Asp Ile His Gln Arg Asp Ala Glu Asn Thr
   225                 230                 235                 240
   Met Arg Glu Leu Ala Val Arg Glu Gly Ile Phe Cys Gly Val Ser Ser
                   245                 250                 255
   Gly Gly Ala Val Ala Gly Ala Leu Arg Val Ala Lys Ala Asn Pro Asp
               260                 265                 270
   Ala Val Val Val Ala Ile Ile Cys Asp Arg Gly Asp Arg Tyr Leu Ser
           275                 280                 285
   Thr Gly Val Phe Gly Glu Glu His Phe Ser Gln Gly Ala Gly Ile
       290                 295                 300
<210> 11
   <211> 34
   <212> DNA
   <213> Artificial Sequence
<220>
   <223> Description of Artificial Sequence: primer
<400> 11
   agctgagtcg accgcctcgt tatcatccaa aatc 34
<210> 12
   <211> 33
   <212> DNA
   <213> Artificial Sequence
<220>
   <223> Description of Artificial Sequence: primer
<400> 12
   agctgagcat gcactaattt tccttgcgga ggc 33
<210> 13
   <211> 32
   <212> DNA
   <213> Artificial Sequence
<220>
   <223> Description of Artificial Sequence: primer
<400> 13
   agctgagcat gctgatggcg gcagcacact gc 32
<210> 14
   <211> 33
   <212> DNA
   <213> Artificial Sequence
<220>
   <223> Description of Artificial Sequence: primer
<400> 14
   agctgatcta gattcactca acctatcagg cgc 33
<210> 15
   <211> 35
   <212> DNA
   <213> Artificial Sequence
<220>
   <223> Description of Artificial Sequence: primer
<400> 15
   agctgagtcg acgtgagtac attagaacaa acaat 35
<210> 16
   <211> 33
   <212> DNA
   <213> Artificial Sequence
<220>
   <223> Description of Artificial Sequence: primer
<400> 16
   agctgatcta gaagtctccg atgctattaa tcc 33
<210> 17
   <211> 34
   <212> DNA
   <213> Artificial Sequence
<220>
   <223> Description of Artificial Sequence: primer
<400> 17
   agctgagtcg actccttaac tgtatgaaat tggg 34
<210> 18
   <211> 33
   <212> DNA
   <213> Artificial Sequence
<220>
   <223> Description of Artificial Sequence: primer
<400> 18
   agctgagcat gcccagcctg tttacgatga tcc 33
<210> 19
   <211> 33
   <212> DNA
   <213> Artificial Sequence
<220>
   <223> Description of Artificial Sequence: primer
<400> 19
   agctgagtcg acatgtcgcg aaaagatggg gtg 33
<210> 20
   <211> 33
   <212> DNA
   <213> Artificial Sequence
<220>
   <223> Description of Artificial Sequence: primer
<400> 20
   agctgatcta gagtttgttc tggccccgac atc 33
<210> 21
   <211> 33
   <212> DNA
   <213> Artificial Sequence
<220>
   <223> Description of Artificial Sequence: primer
<400> 21
   agctgagtcg acatgtcgtg tgaagaactg gaa 33
<210> 22
   <211> 21
   <212> DNA
   <213> Artificial Sequence
<220>
   <223> Description of Artificial Sequence: primer
<400> 22
   atcaccgccg cttcaccaac g 21
<210> 23
   <211> 21
   <212> DNA
   <213> Artificial Sequence
<220>
   <223> Description of Artificial Sequence: primer
<400> 23
   cgttggtgaa gcggcggtga t 21
<210> 24
   <211> 33
   <212> DNA
   <213> Artificial Sequence
<220>
   <223> Description of Artificial Sequence: primer
<400> 24
   agctgatcta gaatagatga ttacatcgca tcc 33
<210> 25
   <211> 33
   <212> DNA
   <213> Artificial Sequence
<220>
   <223> Description of Artificial Sequence: primer
<400> 25
   agctgagtcg acatggcaaa ggtatcgctg gag 33
<210> 26
   <211> 35
   <212> DNA
   <213> Artificial Sequence
<220>
   <223> Description of Artificial Sequence: primer
<400> 26
   agctgatcta gattacagca gacgggcgcg aatgg 35